# EUROPEAN PATENT APPLICATION

(11) **EP 0 614 672 A1**
(43) Date of publication of application: **14.09.1994**
(21) Application number: 93121122.1
(22) Date of filing: 30.12.1993
(51) Int. Cl.: A61L 17/00, A61L 31/00, A61L 27/00, A61L 15/26

(54) **Biocompatible medical devices**

(30) Priority: 31.12.1992 US 999520
(71) Applicant: United States Surgical Corporation, Norwalk, Connecticut 06856 (US)
(72) Inventor: Gruskin, Elliott A., Killingworth, CT 06419 (US)
(74) Representative: Marsh, Roy David

(57) **Abstract**

A medical device or article comprising a synthetic polymer which is biocompatible, the synthetic polymer comprising a lactic and glycolic acid polyester copolymer in which each successive lactic acid and glycolic acid monomeric unit in the polyester copolymer has been individually and specifically determined.

## Description

### BACKGROUND OF THE INVENTION

### Field of the Invention

The present invention relates to biocompatible medical devices such as sutures and the like which also may be absorbable and to methods of making them.

### Description of the Background Art

The advantages of absorbable materials in surgical applications are universally appreciated. The traditional naturally derived suture, known as "catgut," is formed from collagenous material obtained from sheep or beef intestine. More recently, synthetic absorbable sutures of varying chemical composition have been developed.

A number of synthetic polymers have been described for use in making sutures and other bioresorbable medical devices. Effective synthetic absorbable sutures, as well as other medical devices such as haemostatic aids, intraosseous implants, slow-release drug delivery systems, and tissue regeneration devices including nerve channels, sperm ducts, vascular graphs, Fallopian tube ducts and the like, must satisfy a number of biological, physical and chemical requirements. Among these requirements are that the material be bioresorbable, non-carcinogenic, non-antigenic, and non-toxic.

Further, satisfactory bioresorbable polymers for medical applications need to have appropriate mechanical properties including flexibility, tensile strength, dimensional stability, should be sterilizable and absorbable by living tissue at a uniform rate. With respect to sutures, flexibility, adequate straight tensile and knot strength and the capability of being properly and easily tied in surgical knots are particularly desirable characteristics.

Various synthetic polymers have been proposed for use in the fabrication of sutures and other medical devices. Of particular interest are homopolymers and especially copolymers of lactic acid and glycolic acid. Such copolymers have been developed in an attempt to combine the characteristics of both compounds and extend the range of polymer properties and rates of hydrolysis. For example, poly-L-lactic acid is hydrolyzed more slowly than polyglycolic acid and copolymers of the two acids can be made to hydrolyze at intermediate rates. Polymers of this type, and their use in the preparation of synthetic absorbable sutures, are disclosed, for example, in U.S. Patent Nos. 2,703,316, 3,468,853, 3,565,869, 3,636,956, 4,137,921, 4,744,365, 4,839,130 and 5,124,103. Improved braided sutures, which may be composed of lactic acid and glycolic acid copolymers, are described in U.S. Patent Nos. 5,019,093 and 5,037,429.

The use of lactic acid and glycolic acid copolymers in the manufacture of molded medical devices such as, for example, staples or clips is described in U.S. Patent No. 4,523,591, which describes important and desirable properties for such molded articles. That patent also discloses procedures for injection molding, and other suitable molding techniques are known and employed in the art.

U.S. Patent No. 3,736,646 discloses sterile synthetic copolymers containing lactic acid and glycolic acid having enhanced tissue absorption and solubility in organic solvents. That patent also contains reference to a number of other U.S. patents and publications which describe various approaches to the manufacture and use of synthetic polymeric sutures formed from lactic acid and glycolic acid.

Methods of preparing polymers of lactic acid and glycolic acid are described in the patents referred to above. These traditional chemical synthetic methods typically involve the use of a polymerization catalyst which, when combined with appropriately prepared monomer under specified atmospheric and temperature conditions, catalyses the formation of the polymer.

Of course, the way in which a polymer, and especially a copolymer, is made will affect the working characteristics of the resulting suture or other medical device. For example, U.S. Patent No. 5,066,772, which discloses copolymers of recurring units derived from carbonates, lactides and glycolides, discloses copolymers which can be random copolymers or block copolymers, depending upon the properties desired. Random copolymers are disclosed as preferred where soft, pliable and relatively fast bioresorbable materials are required. Block copolymers are disclosed as preferred where hard, crystalline and relative slow bioresorbing materials are required. The patent contains an extensive description of block copolymers and the manner in which the selection of repeating block units may affect properties of the copolymer such as elasticity, modulus, pliability, hardness, softness, crystallinity and bioresorption rate.

U.S. Patent No. 4,137,921 discloses a two-stage polymerization process for the preparation of lactic acid and glycolic acid copolymers. The first stage involves a random copolymerization of optically active lactic acid and glycolic acid monomer by conventional means. A second stage consists of further polymerization of the first stage polymer with additional lactic acid and glycolic acid monomer.

One drawback of traditional synthetic methods of producing polymers, such as those set forth in the U.S. patents referred to above, is that they often involve extreme reaction conditions. These include temperatures as high as 180°C for extended periods of time, use of highly volatile organic solvents such as chloroform and toluene, dry nitrogen reaction atmospheres and high vacuum. Further, these methods require the use of catalysts, some of which may be scarce commodities.

Perhaps the most important disadvantage of prior methods for making synthetic polymers is that they do not allow a high degree of control over the ultimate makeup of the polymer. Traditional chemical synthetic methods of making random copolymers, for example, rely upon crude adjustment of starting material ratios that can, at best, produce a polymer falling somewhere within a broad range of desired characteristics. Similarly, known methods of producing block copolymers are relatively crude, and have the additional disadvantage of requiring tedious and expensive chemical reaction steps.

Copolymer formation also is complicated by the fact that the relative rates of reactivity of glycolide and lactide are different. For example, when equimolar amounts of glycolide and lactide are reacted, glycolide is initially more likely to combine with growing chains than is lactide. Consequently, the initial composition of the growing chain contains a predominance of glycolic acid units occasionally and randomly interspersed with short sequences of lactic acid units. As the reaction proceeds, the concentration of lactide contained in the mixture increases relative to glycolide, and the ratio of glycolic acid units to lactic acid units forming the chain becomes more equal. As the reaction nears completion, most available glycolide has polymerised and the relative amount of lactide is high. Consequently, a larger number of lactic acid units are likely to come together and polymerize.

One consequence of this stoichiometric effect is that the first portion of the copolymer chain is likely to contain a predominance of glycolic acid units, and the end portion of the chain is likely to contain a predominance of lactic acid units. Random sequences generated by the synthesis of poly(lactide-co-glycolide) result in the formation of heterogeneous polymers, i.e., no two polymeric chains are likely to be identically duplicated. Consequently, the physical and chemical properties of such copolymers have been difficult to predict or control with a high degree of precision.

Obviously, optimal control of the properties of a synthetic copolymer material would be attained where the identity of each successive co-monomeric unit was individually and specifically determined from the very outset of the process. It can readily be seen that this would allow an exquisite degree of control, leading to singularly improved biocompatible and absorbable sutures and other medical devices. However, no such method has been described.

Accordingly, it is an object of the present invention to provide improved methods of making lactic acid and glycolic acid copolymers which allow each successive lactic acid or glycolic member of a polymeric chain to be individually and specifically specified. It is another object of the present invention to provide biocompatible and absorbable sutures and other medical devices comprised of lactic acid and glycolic acid copolymers made according to the methods of the invention.

It is a further object of the present invention to allow for the incorporation of individual or multiple amino acids into the polymers made according to the methods of the invention. These can include short or long lengths of amino acids which may have desirable bioactive characteristics. Short or long amino acid sections incorporated into the polymers of the invention may, for example, allow cell attachment, act as growth factors, or prevent thrombosis. Thus, another object of the invention is to provide synthetic copolymer compositions having incorporated therein one or more bioactive elements.

These and other objects of the present invention will be apparent to those of skill who appreciate and understand the teachings of the present specification, set forth in the following description.

### SUMMARY OF THE INVENTION

The present invention is directed to biocompatible medical devices, including sutures, produced from polyesters formed by novel synthetic methods for the template driven synthesis of lactic acid and glycolic acid copolymers of defined sequence. The biocompatible medical devices of the invention also may be absorbable. The novel synthetic methods of the invention allow each successive lactic acid or glycolic acid member of the copolymer to be individually specified: This capability provides an unprecedented degree of control over the design and properties of the copolymer product. As a result of the present invention, greatly improved sutures and other medical devices can now be developed and produced.

### BRIEF DESCRIPTION OF THE DRAWINGS

Figure 1A schematically illustrates a monofilament suture manufacturing operation which is especially suitable for producing larger size sutures employing the polymers of the invention.

Figure 1B schematically illustrates a monofilament suture manufacturing operation which is especially suitable for producing smaller size sutures employing the polymers of the invention.

Figure 2 illustrates a suture employing the polymers of the invention.

Figure 3 is a schematic of the generation of a template for *in vitro* translation.

Figure 4 is a schematic showing the general structure of a template for *in vitro* translation.

Figure 5 illustrates the deamination of tRNA-alanine and tRNA-glycine to their respective a-hydroxyael analogs.

Figure 6 shows the chemical structure of a polyester of defined sequence I according to the present invention.

### DESCRIPTION OF THE PREFERRED EMBODIMENTS

Reference is made in the specification to various methodologies known to those of skill in the art. Publications and other materials setting forth such known methodologies to which reference is made are incorporated herein by reference in their entireties as though set forth in full.

Reagents and the like to which reference is made in the following description and examples are obtainable from commercial sources, unless otherwise noted.

The present invention allows, for the first time, exquisitely precise control over the sequential arrangement of poly(lactide-co-glycolide). One important consequence of this is to allow the design of greatly improved biocompatible and absorbable medical devices. This advantage follows from the ability, previously unknown in this art, to precisely and reproducibly control the rate of hydrolysis of the copolymer.

Those of skill will appreciate that the rate of hydrolysis of a glycolic acid-glycolic bond is greater than the rate of hydrolysis of lactic acid-glycolic acid bond which is greater than the rate of hydrolysis of a glycolic acid-lactic acid bond which is greater than the rate of hydrolysis of a lactic acid-lactic acid bond. Thus, in the copolymer segment: 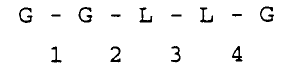
wherein glycolide is oriented to provide a hydroxy terminus on the left-most portion of the segment, i.e., HOCH2CO2CH2...COOH, the order of hydrolysis is 1>4>2>3, i.e., 1 is fastest and 3 is slowest. Therefore, an engineered arrangement of sequential units by means of the present invention will allow precise control over the rate at which a copolymer produced according to the methods of the invention hydrolyzes.

Polyesters having predetermined primary sequence made in accordance with the present invention are suitable for use in a variety of applications. By varying the sequence and length of the polymer, the physical and chemical properties of the polymers can be engineered to meet predefined specifications.

The speed with which a sequential polyester degrades in an environment is based, in part, upon the rate of hydrolysis of ester bonds in the polymer chain. The present invention allows the rate of hydrolysis to be tailored in predictable fashion based upon sequence. Indeed, the precise nature of the copolymers produced according to the present invention allows, *inter alia*, the rate of hydrolysis to be more predictable than possible for prior random copolymers. For example, by means of the present invention, the end portions of a sequential copolymer now may be engineered to hydrolyze more quickly than the central portion of the copolymer chain.

In accordance with the present invention, thermoplastic elastomers may be constructed. Thermoplastic elastomers are multiphase compositions in which the phases are intimately dispersed. The present invention allows thermoplastic elastomers to be constructed by sequential addition of appropriate monomers to fob hard and soft segments within the polymer. In addition, the polymers of the present invention may be combined with conventional polymers known to provide soft segments (such as, for example, polymers formed from epsilon caprolactone, trimethylene carbonate, dioxanone or combinations thereof) or with conventional polymers known to provide hard segments (such as, for example, homopolymeric segments of glycolic or lactic acid).

Sequential polyesters according to the present invention also allow more crystalline structures to be produced. Exquisite control over the chain sequence allows steric regularity to be achieved. Thus, while prior poly(lactide-co-glycolide) polymers containing 25 to 75 mole percent glycolide are amorphous, copolymers containing between 25 to 75 mole percent glycolide can be made crystalline.

In this manner, the tensile strength and other physical properties now can be regulated to a high degree. For example, by varying the proportion of crystalline region to amorphous region, properties such as tensile strength and brittleness now may be infinitely varied to suit particular applications.

In accordance with the present invention, oligomers of sequential polyesters may be coupled to prepare larger, higher molecular weight chains of sequential polyesters. This may be accomplished, for example, by bulk polymerization of pentachlorophenol ester monomers. An inert matrix such as CELITE™ diatomaceous earth may be used to enhance removal of the pentachlorophenol during thermal polymerization in vacuum and lead to higher yields and molecular weights. A p-nitrophenol ester may also be used to promote bulk polymerization.

Polyesters having predetermined primary sequence produced in accordance with the present invention may be used to make block copolymers. For example, two or more polymers prepared in accordance with the present invention may be used as the blocks and joined to form a block copolymer having highly uniform characteristics. Alternatively, one or more polymers prepared in accordance with the present invention may be combined with the polymers prepared by other techniques to form block copolymers or a polymer which has a biosynthetically prepared polyester of predetermined monomeric sequence as a segment thereof. In addition, polymers prepared in accordance with the present invention may be blended with each other or with polymers prepared by other techniques to provide a composition having desired characteristics. Methods of forming block copolymers, blends thereof, and blends of different polymers are well known in the art.

Useful products made from oligomeric or polymeric polymers of the present invention include fibrous surgical articles such as sutures, prosthetic ligaments, prosthetic tendons, woven mesh, gauze, dressings, growth matrices and the like. Such fibrous surgical articles may be engineered to be made more or less elastic depending upon end use. Portions of a single length of monofilament can be made to hydrolyze at different rates and to be more or less elastic than other portions.

In one presently preferred mode, the polymers of the invention are used to make surgical sutures. The principles applied in designing and constructing sutures are known in the art and are set forth herein in summary form and by reference to known publications. Those of skill will recognize that many of these principles will apply also to the design and construction of other medical devices which may be produced using the polymers of the invention.

Multifilament sutures of the present invention may be made by methods known in the art. Braid constructions such as those disclosed and claimed in U.S. Patent Nos. 5,059,213 and 5,019,093 are suitable for the polyester multifilament suture of the present invention.

Monofilament sutures may be manufactured by methods well known in the art. A suitable process for the manufacture of monofilament sutures of the present invention comprises the operations of melt extruding the polyester resin to provide a monofilament, and stretching the solidified monofilament at a temperature above ambient temperature in water (or other suitable liquid medium) or in air (or other suitable gaseous medium) to provide a stretched monofilament. Optionally, the monofilament may then be annealed to provide the finished suture.

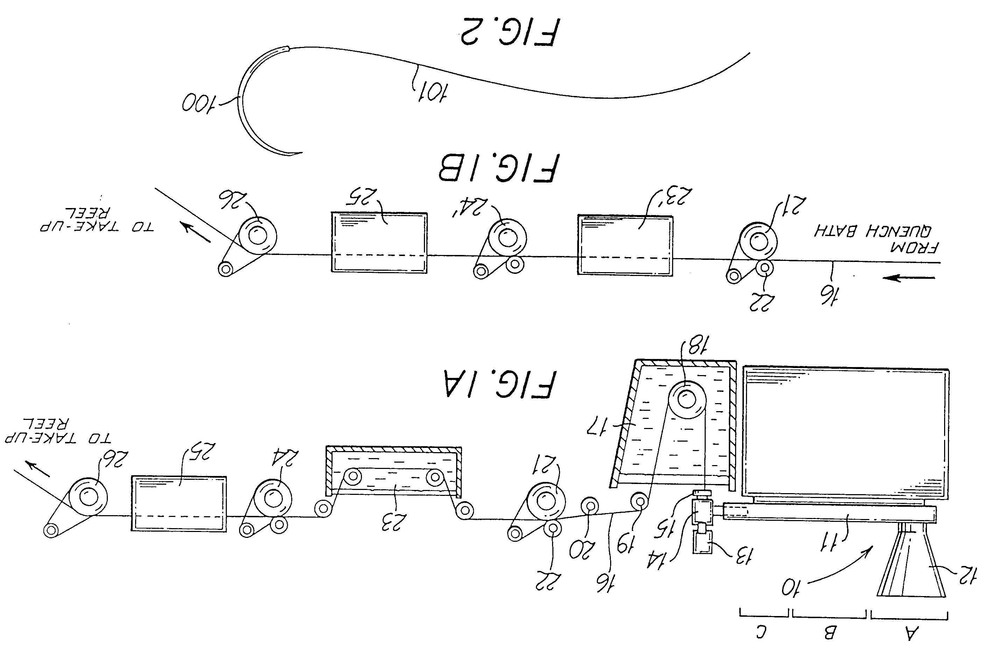

Figure 1A schematically illustrates a monofilament suture manufacturing operation which is especially suitable for producing larger size sutures, e.g., those of sizes 3/0 and larger. Extruder unit 10 is of a known or conventional type and is equipped with controls for regulating the temperature of barrel 11 in various zones thereof, e.g., progressively higher temperatures in three consecutive zones A, B and C along the length of the barrel. Pellets or powder of resin prepared in accordance with the present invention are introduced to the extruder through hopper 12. Any of the polyester compositions of the present invention which are useful for the formation of fibers can be used herein.

Motor-driven metering pump 13 delivers melt extruded resin at a constant rate to spin pack 14 and thereafter through spinneret 15 possessing one or more orifices of desired diameter to provide a molten monofilament 16 which then enters quench bath 17, e.g., containing water, where the monofilament solidifies. The distance monofilament 16 travels after emerging from spinneret 15 to the point where it enters quench bath 17, i.e. the air gap, can vary and can advantageously be from about 0.5 to about 100 cm. If desired, a chimney (not shown), or shield, can be provided to reduce the length of the air gap, e.g. to from 1 to 10 cm, thereby isolating monofilament 16 from contact with air currents which might otherwise affect the cooling of the monofilament in an unpredictable manner.

Monofilament 16 is passed through quench bath 17 around driven roller 18 and over idle rollers 19 and 20. Optionally, a wiper (not shown) may remove excess water from the monofilament as it is removed from quench bath 17. On exiting the quench bath the monofilament is wrapped around a first godet 21 provided with nip roll 22 to prevent slippage which might otherwise result from the subsequent stretching operation. Monofilament 16 passing from godet 21 is stretched, to effect its orientation and thereby increase its tensile strength. In the stretching operation shown in Figure 1A, generally suitable for larger size sutures, e.g., sizes 2 to 3/0, monfilament 16 is drawn through hot water draw bath 23 by means of second godet 24 which rotates at a higher speed than first godet 21 to provide the desired stretch ratio.

In an alternate stretching operation shown in Figure 1B, generally preferred for smaller suture sizes, e.g., sizes 4/0 to 8/0, monofilament 16 is drawn by second godet 24' through hot air convection oven chamber 23' to provide the desired amount of stretch. Following the stretching operation shown in Figures 1A or 1B, monofilament 16 optionally may be subjected to an on-line annealing without shrinkage or relaxation with shrinkage operation as a result of which the monofilament shrinks. In the process of Figures 1A and 1B, on-line annealing with or without relaxation when desired is accomplished by driving monofilament 16 by third godet 26 through second hot air oven chamber 25. For relaxation, the third godet rotates at a slower speed than the second godet thus relieving tension on the filament.

Although not depicted in the Figures, those of skill will appreciate that multiple stretching steps may be used, as are known in the art.

A suture in accordance with the present invention, suture 101, may be attached to a surgical needle 100 as shown in Figure 2 by methods well known in the art. Wounds may be sutured by approximating tissue and passing the needled suture through tissue to create wound closure. The needle preferably is then removed from the suture and the suture tied.

Those of skill will appreciate that other medical articles or devices can be manufactured from the sequential polyesters of the present invention. These include, but are not limited to, solid products, which may be molded or machined, such as orthopedic pins, clamps, screws and plates; clips; staples; hooks; buttons; snaps; bone substitutes such as mandible prostheses; needles; non-permanent intrauterine devices such as spermicides; temporary draining or testing tubes or capillaries; surgical instruments; vascular and ocular implants or supports; vertebral discs; and extracorporeal tubing for, e.g., kidney and heart-lung machines. Also included are fibrillar products, knitted or woven, and including velours, such as burn dressings; hernia patches; absorbent paper or swabs; medicated dressings; facial substitutes; gauze, fabric, sheet, felt or sponge for hemostasis, as, e.g., of the liver or other internal organs; gauze bandages; and dental packs. Other products include flake or powder for burns or abrasions; foam as an absorbable prosthesis; wire substitutes in fixations; and film sprays for prosthetic devices. The sequential polyesters of the present invention may be used alone or in combination with other materials to produce products including those listed hereinabove, as well as combination products such as digestible ion-exchange resins; digestible or time-release devices and drug delivery devices or systems such as pills, patches and pellets; reinforced bone pins, needles, and the like; arterial grafts or substitutes; bandages for skin surfaces; and burn dressings (e.g., in combination with other polymeric films). Nonabsorbable sutures and methods of making them are well known and are described, for example, in U.S. Patent Nos. 3,630,205 and 4,911,165. The biocompatible sequential polyesters of the present invention thus may be combined or blended with the polypropylene compositions of those patents to produce medical articles such as sutures. Presently preferred medical articles include sutures as set forth above, as well as absorbable staples and clips as set forth, for example, in U.S. Patent Nos. 4,523,591, 4,744,365, 4,839,130, 4,844,854, and 5,124,103. These and other non-limiting useful medical articles are known in the art and contemplated as within the scope of the present invention.

Implantable surgical articles made from the polyesters of this invention may be designed to be implanted into patients where the articles are hydrolyzed and absorbed.

It is contemplated that it may be desirable to dye the medical articles of the present invention. For example, a dye may be used to increase visibility of a suture in the surgical field. Dyes known to be suitable for incorporation into medical articles can be used alone or in combination to produce a desired color or shade. Such dyes include but are not limited to Logwood extract, carbon black, and D & C Green No. 6 as described in Marrion, D.M., U.S. Colorants for Food, Drugs, and Cosmetics (1979). Preferably, medical articles such as sutures in accordance with the invention are dyed by adding up to about a few percent dye, such as D & C Green No. 6, to the resin prior to extrusion. Those of skill who appreciate the teachings of the present invention will recognize that detectable moieties also may be incorporated directly into the polymer itself, e.g., *via* a side chain linkage. Such detectable moieties include, but are not limited to, dyes, fluorescers, bioluminescent and chemiluminescent molecules, radionuclides and the like.

Drug delivery devices or systems, as used herein, include any device or article of manufacture which is used to deliver a medicinal agent. The term "medicinal agent" is used in its broadest sense and includes any substance or mixture of substances useful in medicine. Thus, it is understood that a medicinal agent may be a drug, enzyme, peptide, protein, dye, or diagnostic agent such as a detectable moiety which may have no biological activity *per se*.

Examples of various medicinals that can be used in accordance with the present invention include antimicrobials, analgesics, antipyretics, anesthetics, antiepileptics, antihistamines, anti-inflammatories, cardiovascular drugs, diagnostic agents, sympathomimetics, cholinomimetics, anti-muscarinics, antispasmodics, hormones, growth factors, muscle relaxants, adrenergic neuron blockers, anti-neoplastics, immunosuppressants, gastrointestinal drugs, diuretics, steroids and enzymes. It is also intended that combinations of medicinals can be used in accordance with the present invention.

The present invention employs the methods of recombinant genetics in a novel and elegant manner to produce lactic acid and glycolic acid copolymers of specifically defined sequences. By means of this invention, the mechanisms of cellular protein synthesis have for the first time been adapted to the production of lactic acid and glycolic acid copolymers of use in the manufacture of biocompatible and absorbable sutures and other medical devices.

Among the novel and important aspects of the invention is the recognition by the present inventor that a synthetic messenger RNA (mRNA) can be utilized as a template to direct the synthesis not of proteins, as occurs in nature, but of synthetic polyester copolymers of lactic acid and glycolic acid. When the novel approach of the present invention is appreciated, it will be seen that the invention and numerous useful variants of the invention may readily be carried out by those of skill using methods known in the art and as described herein.

Standard reference works setting forth the general principles of recombinant DNA technology include Watson, J.D. et al., Molecular Biology of the Gene, Volumes I and II, The Benjamin/Cummings Publishing Company, Inc., publisher, Menlo Park, CA (1987); Darnell, J.E. et al., Molecular Cell Biology, Scientific American Books, Inc., publisher, New York, N.Y. (1986); Lewin, B.M., Genes II, John Wiley & Sons, publishers, New York, N.Y. (1985); Old, R.W., et al., Principles of Gene Manipulation: An Introduction to Genetic Engineering, 2d edition, University of California Press, publisher, Berkeley, CA (1981); and Maniatis, T., et al., Molecular Cloning: A Laboratory Manual, Cold Spring Harbor Laboratory, publisher, Cold Spring Harbor, NY (1982).

It may be convenient in understanding the invention to set forth definitions of certain terms used herein.

By "cDNA" is meant complementary or copy DNA produced from an RNA template by the action of RNA-dependent DNA polymerase (reverse transcriptase). Thus a "cDNA clone" means a duplex DNA sequence complementary to an RNA molecule of interest, carried in a cloning vector.

By "vector" is meant a DNA molecule, derived from a plasmid or bacteriophage, into which fragments of DNA may be inserted or cloned. A vector will contain one or more unique restriction sites, and may be capable of autonomous replication in a defined host or vehicle organism such that the cloned sequence is reproducible. Thus, by "DNA expression vector" is meant any autonomous element capable of replicating in a host independently of the host's chromosome, after additional sequences of DNA have been incorporated into the autonomous element's genome. Such DNA expression vectors include bacterial plasmids and phages. Preferred for the purposes of the present invention is the lambda gtII expression vector. Also preferred is the commercially available pSPORT plasmid (BRL, Gaithersburg, MD).

By "functional derivative" is meant the "fragments," "analogs," or "chemical derivatives" of a molecule. A "fragment" of a molecule, such as any of the mRNA sequences of the present invention, is meant to refer to any nucleotide subset of the molecule. An "analog" of a molecule is meant to refer to a non-natural molecule substantially similar to either the entire molecule or a fragment thereof.

A molecule is said to be "substantially similar" to another molecule if the sequence of both molecules is substantially the same. Substantially similar molecules will possess similar characteristics. As used herein, a molecule is said to be a "chemical derivative" of another molecule when it contains additional chemical moieties not normally a part of the molecule. Such moieties may improve the molecule's solubility, absorption, biological half life, etc. The moieties may alternatively decrease the toxicity of the molecule, eliminate or attenuate any undesirable side effect of the molecule, etc. Moieties capable of mediating such effects are disclosed, for example, in Remington's Pharmaceutical Sciences, 16th ed., Mack Publishing Co., Easton, Penn. (1980).

Similarly, a "functional derivative" of a nucleotide sequence of the present invention is meant to include "fragments" or "analogues" of the sequence, which may be "substantially similar" in nucleotide sequence, and which encode a molecule possessing similar activity.

A nucleotide sequence encoding the polyesters of the invention may be recombined with vector DNA in accordance with conventional techniques, including blunt-ended or staggered-ended termini for ligation, restriction enzyme digestion to provide appropriate termini, filling in of cohesive ends as appropriate, alkaline phosphatase treatment to avoid undesirable joining, and ligation with appropriate ligases. Techniques for such manipulations are disclosed by Maniatis, T., et al., supra, and are well known in the art.

A nucleic acid molecule, such as DNA, is said to be "capable of expressing" a polyester if it contains nucleotide sequences which contain transcriptional and translational regulatory information and such sequences are "operably linked" to nucleotide sequences which encode the polyester. An operable linkage is a linkage in which the regulatory DNA sequences and the DNA sequence sought to be expressed are connected in such a way as to permit gene expression. The precise nature of the regulatory regions needed for gene expression may vary from organism to organism, but shall in general include a promoter region which, in prokaryotes, contains both the promoter (which directs the initiation of RNA transcription) as well as the DNA sequences which, when transcribed into RNA, will signal the initiation of polyester synthesis. Such regions will normally include those 5'-non-coding sequences involved with initiation of transcription and translation, such as the TATA box, capping sequence, CAAT sequence, and the like.

If desired, a non-coding region 3' to the gene sequence coding for the polyester may be provided by well-known methods. This region may provide transcriptional termination regulatory sequences, such as termination and polyadenylation. Where the transcriptional termination signals are not satisfactorily functional in the expression host cell or system, a 3' region functional in the host cell may be substituted.

Two nucleotide sequences (such as a promoter region sequence and a polyester encoding sequence) are said to be operably linked if the nature of the linkage between the two sequences does not (1) result in the introduction of a frame-shift mutation, (2) interfere with the ability of the promoter region sequence to direct the transcription of the polyester encoding sequence, or (3) interfere with the ability of the polyester encoding sequence to be transcribed by the promoter region sequence. Thus, a promoter region would be operably linked to a polyester encoding sequence if the promoter were capable of effecting transcription of that sequence.

Thus, to express the polyester, transcriptional and translational signals recognized by an appropriate host are necessary.

In a presently preferred embodiment, the present invention utilizes a cell-free translation system to produce polyesters, as is described more fully hereinafter. The present invention also contemplates the expression of polyesters and their functional derivatives in prokaryotic or eukaryotic cells. Preferred prokaryotic hosts include bacteria such as E. coli, Bacillus, Streptomyces, Pseudomonas, Salmonella, Serratia, etc. The most preferred prokaryotic host is E. coli. Other enterobacteria such as Salmonella typhimurium or Serratia marcescens, and various Pseudomonas species may also be utilized. Under such conditions, the polyester will not be glycosylated. The procaryotic host must be compatible with the replicon and control sequences in the expression plasmid. Those of skill will appreciate, of course, that many of the principles which apply to prokaryotic and eukaryotic expression of polyesters according to the invention also will apply to cell-free *in vitro* translation systems.

To express the polyester in a prokaryotic cell (such as, for example, E. coli, B. subtilis, Pseudomonas, Streptomyces, etc.), it is necessary to operably link the polyester encoding sequence to a functional prokaryotic promoter. Such promoters may be either constitutive or, more preferably, regulatable (i.e., inducible or derepressible). Examples of constitutive promoters include the int promoter of bacteriophage lambda, the bla promoter of the beta-lactamase gene of pBR322, and the CAT promoter of the chloramphenicol acetyl transferase gene of pBR325, etc. Examples of inducible prokaryotic promoters include the major right and left promoters of bacteriophage lambda (P_{L} and P_{R}), the trp, recA, lacZ, lacI, and gal promoters of E. coli, the alpha-amylase (Ulmanen, I., et al., J. Bacteriol. 162:176-182 (1985)) and the sigma-28-specific promoters of B. subtilis (Gilman, M.Z., et al., Gene 32:11-20 (1984)), the promoters of the bacteriophages of Bacillus (Gryczan, T.J., In: The Molecular Biology of the Bacilli, Academic Press, Inc., NY (1982)), and Streptomyces promoters (Ward, J.M., et al., Mol. Gen. Genet. 203:468-478 (1986)). Prokaryotic promoters are reviewed by Glick, B.R., (J. Ind. Microbiol. 1:277-282 (1987)); Cenatiempo, Y. (Biochimie 68:505-516 (1986)); and Gottesman, S. (Ann. Rev. Genet. 18:415-442 (1984)).

Proper expression in a prokaryotic cell also requires the presence of a ribosome binding site upstream of the gene-encoding sequence. Such ribosome binding sites are disclosed, for example, by Gold, L., et al. (Ann. Rev. Microbiol. 35:365-404 (1981)).

Eukaryotic hosts include yeast, insects, fungi, and mammalian cells (especially human cells) either *in vivo*, or in tissue culture. Mammalian cells can provide post-translational modifications to polyester molecules including folding or glycosylation, if desired. Mammalian cells which may be useful as hosts include cells of fibroblast origin such as VERO or CHO-K1, or cells of lymphoid origin, such as the hybridoma SP2/O-AG14 or the myeloma P3x63Sg8, and their derivatives. Preferred mammalian host cells include SP2/0 and J558L, as well as neuroblastoma cell lines such as IMR 332, that may provide better capacities for correct post-translational processing. COS cells also are convenient eukaryotic hosts for polyester expression.

For a mammalian host, many possible vector systems are available for the expression of polyesters. A wide variety of transcriptional and translational regulatory sequences may be employed, depending upon the nature of the host. The transcriptional and translational regulatory signals may be derived from viral sources, such as adenovirus, bovine papilloma virus, Simian virus, or the like, where the regulatory signals are associated with a particular gene which has a high level of expression. Alternatively, promoters from mammalian expression products, such as actin, collagen, myosin, etc., may be employed. Transcriptional initiation regulatory signals may be selected which allow for repression or activation, so that expression of the genes can be modulated. Of interest are regulatory signals which are temperature-sensitive so that by varying the temperature, expression can be repressed or initiated, or are subject to chemical regulation, e.g., metabolites.

Yeast also carry out post-translational modifications including glycosylation. A number of recombinant strategies exist which utilize strong promoter sequences and high copy number of plasmids which may be utilized for production of the desired polyesters in yeast. Yeast recognize leader sequences on cloned mammalian gene products and secrete peptides bearing leader sequences (i.e., pre-peptides).

Any of a series of yeast gene expression systems may be used which incorporate promoter and termination elements from the actively expressed genes coding for glycolytic enzymes produced in large quantities when yeast are grown in mediums rich in glucose. Known glycolytic genes can also provide very efficient transcriptional control signals. For example, the promoter and terminator signals of the phosphoglycerate kinase gene may be utilized.

Production of polyesters in insects may be achieved, for example, by infecting the insect host with a baculovirus engineered to express polyesters. Methods of infecting insect hosts using baculoviruses are known to those of skill. Thus, in one embodiment, sequences encoding polyesters according to the invention may be operably linked to the regulatory regions of the viral polyhedrin protein (Jasny, Science 238: 1653 (1987)). Cultured insect cells, or the live insects themselves, which are infected with the recombinant baculovirus, can produce the polyester in amounts as great as 20 to 50% of total protein production.

As discussed above, expression of polyesters in eukaryotic hosts will require the use of eukaryotic regulatory regions. Such regions will, in general, include a promoter region sufficient to direct the initiation of RNA synthesis. Preferred eukaryotic promoters include the promoter of the mouse metallothionein I gene (Hamer, D., et al., J. Mol. Appl. Gen. 1:273-288 (1982)); the TK promoter of Herpes virus (McKnight, S., Cell 31:355-365 (1982)); the SV40 early promoter (Benoist, C., et al., Nature (London) 290:304-310 (1981)); the yeast gal4 gene promoter (Johnston, S.A., et al., Proc. Natl. Acad. Sci. (USA) 79:6971-6975 (1982); Silver, P.A., et al., Proc. Natl. Acad. Sci. (USA) 81:5951-5955 (1984)).

As is widely known, translation of eukaryotic mRNA is initiated at the codon which encodes the first methionine. For this reason, it may be preferable to ensure that the linkage between a eukaryotic promoter and a nucleotide sequence which encodes the polyester or its functional derivative does not contain any intervening codons which are capable of encoding a methionine (i.e., AUG), although those of skill will appreciate that exceptions to this general rule exist, as discussed herein. The presence of such codons results either in a formation of a fusion protein (if the AUG codon is in the same reading frame as polyester encoding nucleotide sequence) or a frame-shift mutation (if the AUG codon is not in the same reading frame).

The polyester encoding sequence and an operably linked promoter may be introduced into a recipient prokaryotic or eukaryotic cell either as a non-replicating DNA (or RNA) molecule, which may either be a linear molecule or, more preferably, a closed covalent circular molecule. Since such molecules are incapable of autonomous replication, the expression of the polyester may occur through the transient expression of the introduced sequence. Alternatively, permanent expression may occur through the integration of the introduced sequence into the host chromosome.

In one embodiment, a vector is employed which is capable of integrating the desired gene sequences into the host cell chromosome. Cells which have stably integrated the introduced DNA into their chromosomes can be selected by also introducing one or more markers which allow for selection of host cells which contain the expression vector. The marker may provide for prototrophy to an auxotropic host, biocide resistance, e.g., antibiotics, or heavy metals, such as copper or the like. The selectable marker gene can either be directly linked to the DNA gene sequences to be expressed, or introduced into the same cell by co-transfection. Additional elements may also be needed for optimal synthesis of single chain binding protein mRNA. These elements may include splice signals, as well as transcription promoters, enhancers, and termination signals. cDNA expression vectors incorporating such elements include those described by Okayama, H., Mol. Cel. Biol. 3:280 (1983).

In one embodiment, the introduced sequence may be incorporated into a plasmid or viral vector capable of autonomous replication in the recipient host. Any of a wide variety of vectors may be employed for this purpose. Factors of importance in selecting a particular plasmid or viral vector are known and include the ease with which recipient cells that contain the vector may be recognized and selected from those recipient cells which do not contain the vector; the number of copies of the vector which are desired in a particular host; and whether it is desirable to be able to "shuttle" the vector between host cells of different species. Prokaryotic vectors include plasmids such as those capable of replication in E. coli (such as, for example, pBR322, ColE1, pSC101, and pACYC 184. Such plasmids are, for example, disclosed by Maniatis, T., et al. (In: Molecular Cloning, A Laboratory Manual, Cold Spring Harbor Press, Cold Spring Harbor, NY (1982)). Bacillus plasmids include pC194, pC221, pT127, etc. Such plasmids are disclosed by Gryczan, T. (In: The Molecular Biology of the Bacilli, Academic Press, NY (1982), pp. 307-329). Suitable Streptomyces plasmids include pIJ101 (Kendall, K.J., et al., J. Bacteriol. 169:4177-4183 (1987)), and Streptomyces bacteriophages such as CHI-C31 (Chater, K.F., et al., In: Sixth International Symposium on Actinomycetales Biology, Akademiai Kaido, Budapest, Hungary (1986), pp. 45-54). Pseudomonas plasmids are reviewed by John, J.F., et al. (Rev. Infect. Dis. 8:693-704 (1986)), and Izaki, K. (Jpn. J. Bacteriol. 33:729-742 (1978)).

Preferred eukaryotic plasmids include BPV, vaccinia, SV40, 2-micron circle, etc., or their derivatives. Such plasmids are well known in the art (Botstein, D., et al., Miami Wntr. Symp. 19:265- 274 (1982); Broach, J.R., In: The Molecular Biology of the Yeast Saccharomyces: Life Cycle and Inheritance, Cold Spring Harbor Laboratory, Cold Spring Harbor, NY, p. 445-470 (1981); Broach, J.R., Cell 28:203-204 (1982); Bollon, D.P., et al., J. Clin. Hematol. Oncol. 10:39-48 (1980); Maniatis, T., In: Cell Biology: A Comprehensive Treatise, Vol. 3, Gene Expression, Academic Press, NY, pp. 563-608 (1980)).

Once the vector or nucleotide sequence containing the construct(s) has been prepared for expression, the vector or nucleotide construct(s) may be introduced into an appropriate host cell by any of a variety of suitable means, including such biochemical means as transformation, transfection, conjugation, protoplast fusion, calcium phosphate-precipitation, and application with polycations such as diethylaminoethyl (DEAE) dextran, and such mechanical means as electroporation, direct microinjection, and microprojectile (biolistic) bombardment (Johnston, et al., Science 240(4858): 1538 (1988)), etc.

After the introduction of the vector, recipient cells are grown in a selective medium, which selects for the growth of vector-containing cells. Expression of the introduced nucleotide sequence(s) results in the production of the polyester, or in the production of a fragment of the polyester. This can take place in the transformed cells as such, or following the induction of these cells to differentiate (for example, by administration of bromodeoxyuracil to neuroblastoma cells or the like).

The sequential polyester produced according to the invention may be isolated and purified in accordance with conventional methods, such as extraction, precipitation, chromatography, affinity chromatography, electrophoresis, or the like.

In one embodiment, the present invention is directed to the use of synthetic messenger ribonucleic acid (mRNA) as a template for the synthesis of defined sequences of lactic acid and glycolic acid copolymers.

mRNA prepared according to the methods of the invention and known in the art can be used to direct defined copolymer synthesis in a cell-free *in vitro* translation system. *In vitro* translation systems are well known in the art, and their use for the incorporation of unnatural amino acids into proteins is described, for example, by Noren, et al., Science 244:182 (1989); Robertson, et al., Nucleic Acids Res. 17(23):9649 (1989); Anthony-Cahill, et al., TIBS [VOL]:400 (1989); Robertson, et al., J. Am. Chem. Soc. 113:2722 (1991); Mendel, et al., J. Am. Chem. Soc. 133:2758 (1991); and Ellman, et al., Science 225:197 (1992), the disclosures of which are incorporated herein in full. Thus, for example, transfer RNA (tRNA) can be chemically modified by known methods to carry lactate rather then the cognate amino acid. When the appropriate codon is reached during translation of mRNA, the tRNA-lactate molecule binds to that codon. Therefore, through the specific synthesis of tRNA's for both lactate and glycolate, a cell free system, programmed by a synthetic mRNA, can be used according to the present invention to synthesize copolymer of lactate and glycolate as defined sequences.

In a preferred embodiment of the invention, chemical acylation may proceed with the generation of truncated tRNA that recognize stop codons, and are therefore termed herein "suppressor tRNA" (abbreviated "Sup-tRNA"). Sup-tRNA generally will lack the two 3' nucleotides: CpA. In separate reactions, CpA may be acylated with the nonamino acid (**X**), employing well known acylation methods. The resulting CpA-**X** may then be enzymatically ligated onto the Sup-tRNA to generate the mature tRNA: Sup-tRNA-**X**. An RNA template may be generated that has the appropriate stop codon within the open reading frame that matches the anticodon in the Sup-tRNA-**X**. An *in vitro* translation system may be used, such that **X** is incorporated at the template directed site.

Truncated tRNA molecules may be synthesized by known methods, for example, by employing modifications of the methods described by Noren, et al., (1989), Robertson, et al., (1991), and Ellman, et al., (1992), incorporated herein in full. In a preferred embodiment, the gene for yeast tRNA^{phe} may be cloned into a M13 type phage vector using well known cloning methods. The anticodon loop may be altered to recognize stop codons using methods well known in the art, such as, for example, oligonucleotide site directed mutagenesis. Since there are three stop codons in the genetic code, two may be used to encode lactate and glycolate, and the third is reserved for the actual translation stop codon. In a presently preferred embodiment, UAA encodes lactate, and UAG encodes glycolate. The remaining stop codon, UGA, will be used for translation termination.

Those of skill will recognize that it may be desirable to truncate the two 3' nucleotides (CpA) of the tRNA^{phe}, using well known methods, such as mutagenesis as described herein and as well known in the art. The gene for the truncated Sup-tRNA^{phe} may be cloned by known methods into an appropriate vector downstream of a suitable promoter. Examples of suitable promoters according to the invention include the T7 or T4 RNA polymerase promoters. An *in vitro* transcription reaction as described herein may be used to generate workable quantities of the truncated Sup-tRNA^{phe}.

The CpA dinucleotide may be synthesized according to the present invention in large quantities employing known methods. Presently preferred is the use of a solid phase Automated DNA synthesizer according to methods known in the art. Chemical acylation of the dinucleotide CpA may be performed by known methods. Presently preferred is the method of Noren, et al. (1989) supra. Briefly, the exocyclic amine of Cytidine is protected with orthonitrophenylsulfenyl chloride (NPS-CL). Since there is no reactive amine on either glycolate or lactate, there is no need for any protection reactions. Glycolate and lactate are coupled to the 2' or 3' hydroxyl group of the Adenine (the 2' and 3' acylations rapidly interconvert). The coupling reaction is carried out with N,N'-carbonyldimidazole as an activating reagent. After the coupling reaction the NPS protecting groups on the Cytidine are removed with aqueous thiosulfate. The result is CpA-lactate and CpA-glycolate.

Purified CpA-lactate and CpA-glycolate may be ligated onto Sup-tRNAₚₕₑ employing well known methods, such as, for example, by the use of the enzyme T4-RNA ligase. The resulting Sup-tRNA^{phe}-lactate and Sup-tRNA^{phe}-glycolate may be purified by any known methods, including but not limited to column chromatography.

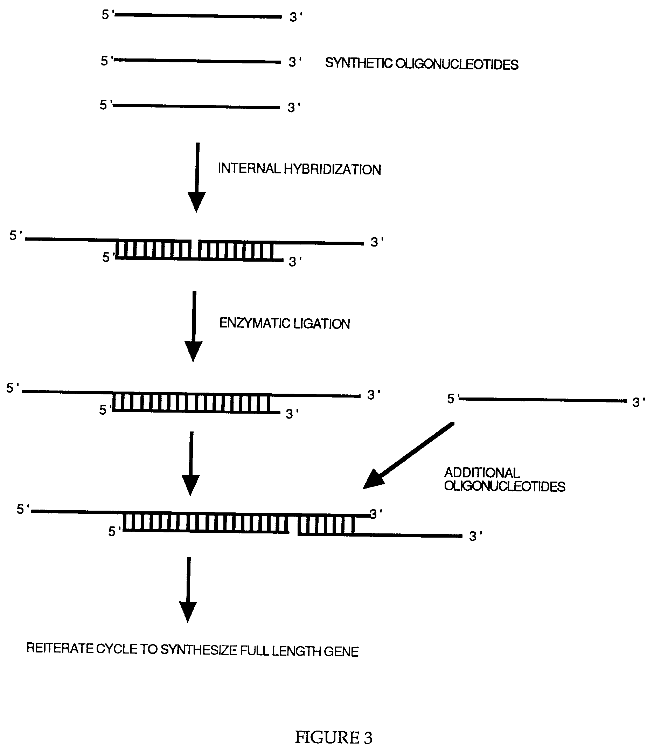

The temp]ate for the *in vitro* translation system according to the present invention may be synthesized from oligonucleotides Oligonucleotides of defined sequences may be synthesized by known methods including, but not limited to, automated DNA synthesis. A scheme of overlapping internal hybridizations may be used to generate a complete template, as depicted in Figure 3. In one embodiment, the template may be ligated by known methods into an appropriatevector downstream from and in frame with a protein coding segment. The purpose of the peptide segment in this presently preferred embodiment is to efficiently initiate translation. The junction between the amide and polyester segment will be chosen to permit rapid, efficient, and specific post-translational cleavage of the polyester segment from the amide leader segment. Non-limiting examples of possible amide-polyester junctions according to the invention include amide regions terminating with: 1) methionine, which may be cleaved with cyanogen bromide, 2) lysine or arginine, which may be cleaved with trypsin, 3) phenylalanine, tryptophan or tyrosine, which may be cleaved by chymotrypsin.

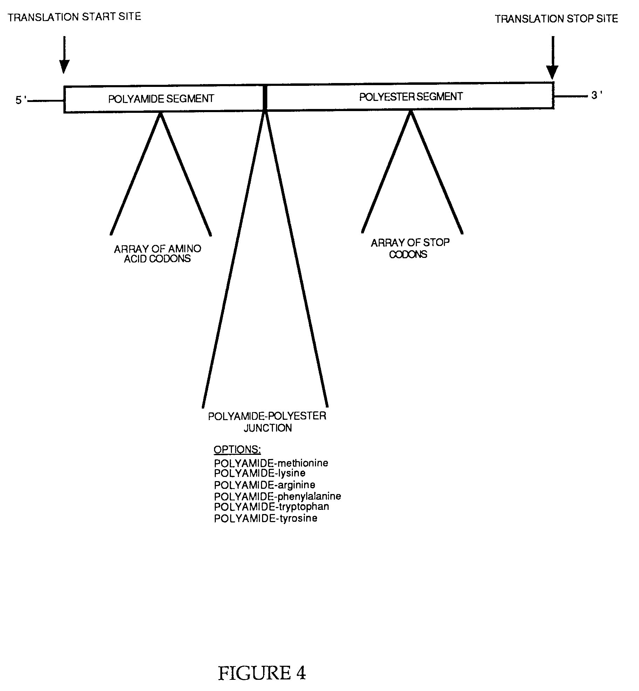

In a preferred embodiment, the sequence of the polyester-encoding template is a series of stop codons recognized by Sup-tRNA^{phe}-glycolate. According to this embodiment, a string of UAA and UAG units will encode the corresponding sequence of lactate and glycolate units. Those of skill having benefit of the teachings of the present specification will recognize that any combination of triplet codons may be chosen to define any ordered sequence of lactate and glycolate units. At the completion of the sequence, the stop codon UAG, which in the presently preferred embodiment is not suppressed, is used to terminate translation. Thus, the template so produced according to the invention will have the general structure shown in Figure 4.

In a preferred embodiment of the invention, an E. coli *in vitro* translation system may be employed. E. coli amino-acyl tRNA transferase cannot amino-acylate yeast tRNA, which, as described above, can be the origin of the Sup-tRNA^{phe}-glycolate. Thus, it will be appreciated that once the Sup-tRNAphe-lactate and Sup-tRNAphe-glycolate participate in a translation cycle, the free Sup-tRNA^{phe} will not be amino-acylated with phenylalanine and thus interfere with polyester synthesis. In addition, the E.coli strain used to generate the *in vitro* translation extract is a recombinant strain that does not express tRNA for the two stop codons used to encode glycolate and lactate. Therefore, native tRNA will not compete with Sup-tRNA^{phe}-glycolate for recognition of stop codons.

The translation extract may be used according to the invention to synthesize polymers in conjunction with the artificial template. Thus, for example, free amino acids, ATP, GTP, Sup-tRNA^{phe}-lactate and Sup-tRNA^{phe}-glycolate, and template may be added to the E. coli extract in order to allow translation to occur. Translation may be terminated by any appropriate means as are well known in the art, for example, by the addition of a detergent.

As used herein, "Releasing Factors" are proteins necessary to terminate translation at stop codons. Two of these factors are designated RF 1 (which recognizes UAA and UAG) and RF 2 (which recognizes UAA and UGA). In a preferred embodiment of the invention, an E. coli translation system may be employed based upon RF mutants such that competition between chain extension and termination is minimized. Since the Releasing Factors have UAA in common, it is presently preferred to make the E. coli cell-free translation system from E. coli that do not express RF 1. By so doing, competition for the termination of translation at UAG codons may be prevented or minimized.

The amide-polyester polymer may be purified by known methods, such as, for example, column chromatography and phase separative procedures. The purified polymer may be processed by cleavage steps such as those described herein to separate the amide from the polyester segment. If desired, the polyester segment may be further purified by methods known in the art, such as extraction methods or additional column chromatography.

Methods by which tRNA^{phe}-phenylalanine may be deaminated to tRNA^{phe}-phenyllactyl are known and are described, for example, by Fahnestock and Rich, *Science***173**:340 (1971). The resulting a-hydroxyael analog of tRNA^{phe}-phenylalanine has been shown to be active in an *in vitro* translation system to generate polyesters. In a presently preferred embodiment of the invention, this strategy may be applied to the production of Sup-tRNA-lactate and Sup-tRNA-glycolate. Instead of generating Sup-tRNA by an *in vitro* transcription system, and then ligating CpA-lactate or CpA-glycolate to the tRNA, Sup-tRNA^{gly}-glycolate and Sup-tRNA^{ala}-lactate may be produced directly in a fermentation process. These tRNA species are then translated *in vitro* using templates and translation systems as described herein.

Thus, for example, genes for yeast tRNA^{gly} and tRNA^{ala} may be cloned onto an appropriate cloning vector using methods known to those of skill. Appropriate cloning vectors may be obtained from commercial sources, and include, for example, the M13 type phage vector. Once the genes have been inserted into the cloning vector, new anticodons that recognize stop codons (Sup-tRNA^{gly} and Sup-tRNA^{ala}) may be inserted by known methods, such as, for example, site directed mutagenesis. The resulting mutagenized tRNA will thus contain new anticodons that recognize stop codons (Sup-tRNA^{gly} and Sup-tRNA^{ala}). In a presently preferred embodiment, the stop codons for tRNA^{ala} and for tRNA^{gly} may be UAA and UAG, respectively. The new tRNA genes may be cloned onto an appropriate expression vector using known methods. Preferably, the new tRNA genes will be cloned onto the chosen expression vector downstream of an appropriate promoter. In a preferred embodiment, the promoter will be an inducible promoter. The resulting expression vector or plasmid may be used to transform an appropriate host cell, such as, for example, yeast. The resulting recombinant yeast strain will be capable of producing quantities of Sup-tRNA^{gly}-glycine and Sup-tRNA^{ala}-alanine.

Thus, in a preferred embodiment, the cultures of the recombinant yeast cells may be grown to near confluence employing known culture methods. If necessary or desirable, a protein synthesis inhibitor may be added and the inducible tRNA genes activated. The yeast will then express the tRNA^{gly} and tRNA^{ala}, which are aminoacylated with the appropriate amino acid. The resulting Sup-tRNA^{gly}-glycine and Sup-tRNA^{ala}-alanine may be purified by standard techniques.

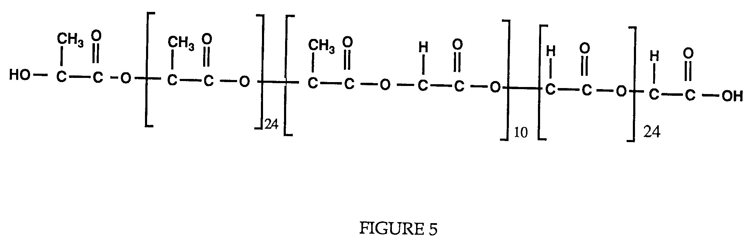

After purification, the Sup-tRNA^{gly}-glycine and Sup-tRNA^{ala}-alanine may be deaminated by known methods, such as described, for example, by Fahnestock and Rich (1971) (supra). Thus, according to this presently preferred method, purified Sup-tRNA^{gly}-glycine and Sup-tRNA^{ala}-alanine are incubated in 0.25 M sodium acetate, 0.01 M magnesium acetate, 1M NaNO₂ at 24°C. The pH is maintained at pH 4.3 with acetic acid. These reaction conditions result in the deamination of the amino acids on the tRNA to the a-hydroxyael analogs, as shown in Figure 5. The resulting Sup-tRNAgly-glycolate and Sup-tRNA^{ala}-lactate may be purified by known methods, such as, for example, column chromatography.

The initiator codon for translation will be selected by the skilled artisan based upon known initiation principles and an appreciation of the teachings of the present invention. For example, polyester synthesis may be initiated by a native E. coli MET-tRNAf. In this case, the resulting polyester has a methionine as the first residue. This first methionine may, if desired, be deleted from the polyester by known methods.

In a presently preferred embodiment, a synthetic initiator tRNA may be synthesized that recognizes the AUG codon, but carries a lactate instead of methionine. A truncated version of the tRNAf may be generated that does not have the CpA dinucleotide at the 3' termini. A synthetic CpA dinucleotide may be synthesized and coupled with lactate to form CpA-lactate. The CpA-lactate may be ligated onto the 3' termini of the truncated tRNAf. The resulting Lactate-tRNAf initiates translation by recognizing the AUG codon. Thus, translation is initiated with lactate rather than methionine. Those of skill will appreciate that where, for example, an E. coli *in vitro* translation system is to be used, the starting tRNAf must be E. coli so that the initiation factors in the E. coli *in vitro* translation system will recognize it.

The invention also relates to nucleotide sequences which encode a fusion product or chimera comprising a polyester or fragment thereof and a detectable enzyme such as beta-galactosidase, or any desired homologous or heterologous protein or peptide. Methods for producing fusion proteins are taught, for example, Bai, D.H., et al., J. Biol. Chem. 261:12395-12399 (1986), or Huynh, T.U., et al., "Construction and Screening cDNA Libraries in lambda-gt10 and lambda-gt11," in DNA Cloning Techniques: A Practical Approach, D. Glover (ed.), IRL Press, Oxford, 1985, pp. 49-77.

The polyester, functional derivative thereof, or fusion protein comprising polyester or fragment thereof and a detectable enzyme or desired protein or peptide may be isolated according to conventional methods known to those skilled in the art. For example, the cells may be collected by centrifugation, or with suitable buffers, lysed, and the protein isolated by column chromatography, for example, on DEAE-cellulose, phosphocellulose, polyribocytidylic acid-agarose, hydroxyapatite or by electrophoresis or immunoprecipitation

Preferred cell free systems of the invention will be derived from E. coli, which is well characterized and which is thus a convenient model. The choice of other prokaryotic and eukaryotic derived cell free systems will be routinely made by those of skill.

Where an E. coli derived cell free system is employed according to the invention, yeast tRNA molecules would be chosen since it has been demonstrated that yeast tRNA's are not recognized by E. coli amino acyl tRNA synthetases (the enzymes in the cell free system that will charge tRNA's with the cognate amino acids). Thus, if an E. coli cell free protein synthesis system is used with yeast tRNA's, the yeast tRNA's would not be charged with any amino acids, such that polymer synthesis would proceed without interference.

Alteration of the yeast tRNA molecules to carry modified lactic acid and glycolic acid is carried out by routine chemical methods, such that the 5' phosphate terminus is shortened by two ribonucleotides. In native tRNA, the two nucleotides are always **C**p**A**.

In a separate reaction, a ribonucleotide dimer, **C**p**A**, would be chemically modified such that a lactate or glycolate is covalently attached to the ribose moiety at the 2' position of the **A** ribonucleotide. The resulting **C**p**A**-Lactate and **C**p**A**-Glycolate products are then enzymatically linked to appropriate tRNA molecules through the use of T4 RNA ligase.

The mRNA used to program the cell free system according to the invention may consist of repeating units of two triplet codons. One codon is recognized by tRNA-Lactate and the other by tRNA-Glycolate. The order of the codons in the mRNA determines the order of the lactate and glycolate units. Since initiation of the synthetic pathway is a complex process which involves a series of reactions centered on a specific start codon, it may be necessary or desirable to start copolymer synthesis with a short segment of mRNA sequence that codes for polypeptide. Once translation is initiated, and a short segment of polypeptide is produced, the ribosome will reach the beginning of the lactate and glycolate codons. Thus, a chimeric polymer will be generated consisting of a short polyamide section, contiguous with a larger polyester segment. By incorporating a methionine at the end of the polyamide section, the polyester can be liberated by treatment of the copolymer with cyanogen bromide, which specifically cleaves polyamides at the carboxyl side of methionines, as is known in the art.

In another method according to the present invention, longer ribonucleotides, consisting of the first 10 or so bases of the tRNA from the 5' terminus, are covalently linked by known methods to lactate or glycolate. In native tRNAs this 10 base region base-pairs with corresponding ribonucleotides, forming an RNA-RNA duplex. The resulting product consists of lactate and glycolate oligoribonucleotides. Mature tRNA's are generated by hydrogen bonding of the lactate and glycolate oligoribonucleotides to the matching bases on the tRNA's. This manipulation bypasses the ligation step described above catalyzed by T4 RNA ligase.

In a cell free system according to this embodiment, excess lactate or glycolate oligoribonucleotides are reacted with a limiting concentration of precursor tRNA molecules. During the course of the translation reaction, the temperature is cycled such that at low temperature the lactate or glycolate-oligoribonucleotides anneal to the precursor tRNA's and participate in polymer chain elongation. At high temperatures, the base pairing between the oligoribonucleotides (that have given up the lactate or glcolate) and the precursor tRNA's is disrupted, such that when the temperature is cycled down, unused lactate or glycolate-oligoribonucleotides anneal to the precursor tRNA's.

In another embodiment of the invention, lactic acid and glycolic acid copolymers are synthesized by means of an *in vivo* translation system. As in the case of the *in vitro* translation system described above, E. coli is the presently preferred host for such a system. Ribonucleotides are synthesized with sulfodiester rather than the natural phosphodiester bonds by means known in the art. The resulting ribonucleotides will be neutrally charged, which will allow them to pass through the E. coli cell membrane.

tRNA precursors for use in an *in vivo* translation system may be encoded by genes with inducible promoters, carried on a plasmid. Inducible E. coli promoters are known in the art, and include those described above. Suitable plasmids for use according to this aspect of the invention include those described above. Further, those of skill will recognize that the anticodons on the tRNA precursors will preferably recognize stop codons, in order to avoid incorporation of native amino acids into the resulting polymers.

In an *in vivo* translation system according to the present invention, the gene that encodes the mRNA template also may be carried on a plasmid under the control of a inducible promoter. The mRNA sequence will preferably comprise the initial polyamide sequence described herein, followed by a sequence that consists of stop codons. The specific order of the stop codons determines the sequence of lactate and glycolate in the resulting polymer.

Those of skill will appreciate that when the promoters are induced, the mRNA, as well as the precursor tRNA's, will be expressed. The addition of lactate and glycolate-ribonucleotides to the incubation medium will result in diffusion of those compounds into the E. coli cells. Cellular polymer synthesis will continue under these conditions until lactate or glycolate-tRNA's are exhausted. At this point, precursor tRNA or exhausted lactate and glycolate-tRNA concentrations will be in excess with respect to the exogenously added lactate and glycolate-oligoribonucleotides, and copolymer synthesis will stop.

Oligonucleotides complimentary to a truncated, tRNA precursor may be synthesized by known methods. Lactate and glycolate may be covalently attached to the oligonucleotide by methods described herein. When the lactate and glycolate oligonucleotides anneal to an appropriate precursor, truncated tRNA, a mature Sup-tRNA-gylcolate or lactate is formed. Lactate and glycolate oligonucleotides are diffused into cells that express the truncated, precursor tRNA's. The lactate and glycolate oligonucleotides anneal to the precursor tRNA's, and polyester synthesis ensues. Important considerations include the stability of the oligonucleotides *in vivo*, and the rate of diffusion of the oligonucleotides into cells. Resistance to nucleases *in vivo* may be accomplished through the use of special nucleotide analogues such as phosphorothioates (Agrawal, et al, Proc. Natl. Acad. Sci. USA, 88, 7595-7599). Diffusion of the oligonucleotides into cells may be enhanced through the use of liposomal encapsulation, electroporation or by synthesizing oligonucleotides with sulfodiester rather than phosphodiester linkages.

A significant feature of the present invention is the inventor's recognition that templates according to the invention may be constructed such that a mixed amide-polyester polymer is translated. In this scheme, the stop codons can be interrupted by non-stop codons which encode amino acids. When these codons are translated, an amino acid will be incorporated into the chain at the appropriate position. Codons for individual amino acids may be incorporated at specific locations. Preferred amino acids include those which may be deaminated to their corresponding alpha hydroxy analogues. Presently preferred amino acids include, but are not limited to, glycine, L-alanine, L-valine, L-isoleucine, L-leucine, L-phenylalanine, L-methionine, L-serine, L-threonine, L-tyrosine, L-tryptophan, L-asparagine, L-glutamine, L-cysteine, L-aspartic acid, L-glutamic acid, L-lysine, L-arginine and L-histidine. Similarly, a series of codons for amino acid segments may be planned into the template. For example, amide portions corresponding to bioactive peptides may be nested within polyester regions. Such peptides may include cell attachment sequences such as R-G-D, growth factors or antithrombogenic peptides.

The plasmid with the template may be used to drive an *in vitro* transcription reaction to produce mRNA. The resulting mRNA may be purified by column chromatography, and used as the template for *in vitro* translation.

Having now described the invention, the same will be more fully understood by those of skill with reference to the following non-limiting examples.

### EXAMPLE I

### TEMPLATE DIRECTED SYNTHESIS OF A POLYESTER WITHOUT A POLYPEPTIDE LEADER SEQUENCE

### I) DESIGNATION OF POLYESTER SEQUENCE:

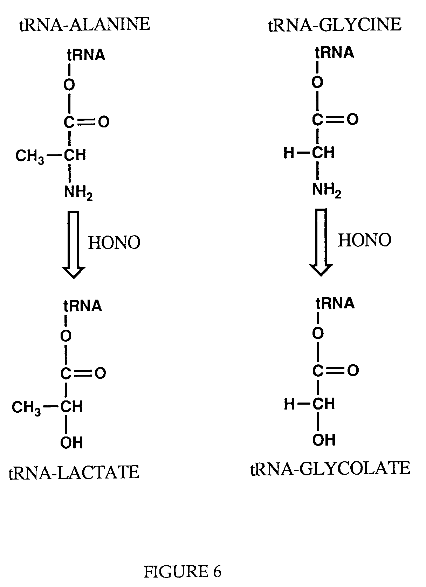

The synthetic method of the present invention allows synthesis of polyesters derived from the a-amino acid analogues of any of the amino acids with the exception of proline. There are three STOP codons: UAA; UAG; and UGA. In the present example, one of these STOP codons is reserved for the STOP signal for the polyester. The remaining two STOP codons thus are available for encoding the monomer units of the polyester. In the present example, lactate and glycolate are encoded by UAA and UAG, respectively. The methods of the invention are utilized to construct a polyester having the following defined sequence:
[Lactate]₂₅-[Lactate-Glycolate]₁₀-[Glycolate]₂₅ I
The chemical structure of I is shown in Figure 6.

### II) DESIGN AND SYNTHESIS OF SYNTHETIC GENE:

A) The polyester coding region: The template sequence for the polyester having sequence I is the sequence of the corresponding codons for each monomer in the polyester chain. Initiation of translation always occurs at an AUG codon. Therefore, the first lactate in the polyester chain is encoded by an AUG codon. This is accomplished through the use of a specially synthesized Met-tRNAf modified to carry lactate instead of methionine, as described herein. Alternatively, a methionine is incorporated in the first position of the polymer chain. The resulting polymer is treated with cyanogen bromide to remove the methionine, as described herein.
   In the present example, the template is synthesized such that the first AUG codon encodes a lactate. Thus, the template, which encodes a polyester having the sequence I, has the following sequence:
   5'-AUG-[UAA]₂₄-[UAA-UAG]₁₀-[UAG]₂₅-UGA-3' II
   Alternatively, if the template is designed such that the first codon is used to encode a methionine, then the template is constructed as follows:
   5'-AUG-[UAA]₂₅-[UAA-UAG]₁₀-[UAG]₂₅-UGA-3' III
B) Stepwise construction of the transcriptional unit:
   1) Construction of the initiator region of the synthetic gene: Since *in vitro* translation of the synthetic mRNA is carried out in an E. coli cell free system, the initiator region must be recognized by E. coli ribosomes. In this example, the initiator region is identical to the E. coli trpA gene. Oligonucleotides of the following sequence are synthesized:
      5'*C*-AGC-ACG-AGG-GGA-AAT-CTG-**ATGTAAT**-GCATG3'
      3'-*TGCAG*-TCG-TGC-TCC-CCT-TTA-GAC-**TACATTA**-C-5' (IV)
      Key:
      Bold = start codon
      Underline = trpA initiator sequence
      Plain text = Sph I adaptors
      Italics = Aat II adaptors
      The oligonucleotides are combined, heated to 90°C, then allowed to cool slowly to room temperature. During cooling, the oligonucleotides anneal to form a double stranded DNA.
      Oligonucleotide IV is ligated into the commercially available pSPORT I plasmid (BRL, Gaithersburg, MD) that has been cleaved with the Aat II and Sph I restriction endonucleases.
      1) Aat II Digestion of pSPORT I Plasmid:
         A 5 mg sample of pSPORT I DNA is treated with 10 units of Aat II (New England Biolabs) for 60 minutes at 37°C in a 50 ml reaction consisting of 50 mM Kacetate, 20 mM Tris-acetate (pH 7.9), 10 mM MgAcetate, 1 mM dithiothreitol (DTT) and 100 mg/ml bovine serum albumin (BSA). The reaction is terminated with the addition of 150 ml 10 mM Tris-HCl (pH 8.0), 1 mM EDTA, followed by extraction with an equal volume of 1:1 buffer saturated phenol:chloroform, isoamyl alcohol (24:1). The aqueous phase is collected, and precipitated by the addition of 20 ml 3 M sodium acetate (pH 5.2) and 400 ml absolute ethanol. The precipitated DNA is collected by centrifugation, washed once in 70% ethanol, then resuspended in 10 ml of water.
      2) Sph I Digestion of pSPORT I Plasmid:
         A 5 mg sample of the Aat II digested pSPORT I DNA is treated with 10 units of Sph I (New England Biolabs) for 60 minutes at 37°C in a 50 ml reaction consisting of 50 mM NaCl, 10 mM Tris-HCl (pH 7.9), 1 mM DTT and 100 mg/ml BSA. The reaction is terminated with the addition of 150 ml 10 mM Tris-HCl (pH 8.0), 1 mM EDTA, followed by extraction with an equal volume of 1:1 buffer saturated phenol:chloroform, isoamyl alcohol (24:1). The aqueous phase is collected, and precipitated by the addition of 20 ml 3 M sodium acetate (pH 5.2) and 400 ml absolute ethanol. The precipitated DNA is collected by centrifugation, washed once in 70% ethanol, then resuspended in 10 ml of water.
      3) Ligation of oligonucleotide IV into Aat II and Sph I digested pSPORT I Plasmid:
         The Aat II and Sph I digested pSPORT I plasmid is combined with 1 mg of annealed oligonucleotide IV. The DNA solution is treated with 10 units of T4 DNA ligase in a 100 ml reaction consisting of 50 mM Tris-HCl (pH 7.8), 10 mM DTT, 1 mM ATP and 100 mg/ml BSA at 15°C for 16 hours. The resulting plasmid is designated pSPORT Ia.
      4) Transformation and selection of pSPORT Ia:
         The ligation reaction is used to transform competent E. coli DH5-a. The transformed E. coli are selected for the presence of plasmid by growing colonies on Lb agar plates with 100 mg/ml ampicilin. Ampicilin resistant colonies are recovered and grown in Lb liquid media with 100 mg/ml ampicilin. Plasmid DNA is purified from the cultures by standard miniprep procedures. The correct construct is verified by the lack of cleavage with Aat II.
      5) Cleavage of pSPORT Ia with Sph I and Hind III:
         A 5 mg sample of pSPORT Ia is digested with Sph I as described above. The Sph I digested DNA is treated with 10 units of Hind III (New England Biolabs) for 60 minutes at 37°C in a 50 ml reaction consisting of 50 mM NaCl, 10 mM Tris-HCl (pH 7.9), 10 mM MgCl₂, 1 mM DTT and 100 mg/ml BSA. The DNA is purified as described above.
      6) Creation of a Fok I site in pSPORT Ia: The pSPORT Ia plasmid is adapted so that the rest of the polyester coding sequence can be constructed downstream of, and in frame with, the ATG start codon. To accomplish this, oligonucleotides that contain a Fok I site are ligated into pSPORT Ia downstream of the ATG start codon. The following oligonucleotides are synthesized:
         -5'-**C**-GCG-CATCC-*A*-3'
         3'**GTACG**-CGC-GTAGG-*TTCGA*-5' (V)
         Key:
         Bold = Sph I adaptors
         Underline = spacer sequence
         Plain text = Fok I site
         Italics = Hind III adapter
         The oligonucleotides having the sequence V are combined, heated to 90°C, then allowed to cool slowly to room temperature. During cooling, the oligonucleotides anneal to form a double stranded DNA. The DNA is ligated into pSPORT Ia that has been cleaved with Sph I and Hind III.
      7) Ligation of Oligonucleotide V into Sph I and Hind III digested pSPORT Ia:
         The Sph I and Hind III digested pSPORT Ia is combined with 1 mg of annealed oligonucleotide V. The DNA solution is treated with 10 units of T4 DNA ligase in a 100 ml reaction consisting of 50 mM Tris-HCl (pH 7.8), 10 mM DTT, 1 mM ATP and 100 mg/ml BSA at 15°C for 16 hours. The resulting plasmid is designated pSPORT Ib. The new sequence in pSPORT Ib is as follows: 
-5′C-AGC-ACG-AGG-GGA-AAT-CTG-ATGTAAT-GCATGC-GCG-CATCC-
A-3′-
-3′-TGCAG-TCG-TGC-TCC-CCT-TTA-GAC-TACATTA-CGTACG-CGC-GTAGG-
TTCGA-5′-
 Key:
         Bold = Fok I site
      8) Transformation and selection of pSPORT Ib:
         The ligation reaction is used to transform competent E. coli DH5-a. The transformed E. coli are selected for the presence of plasmid by growing colonies on Lb agar plates with 100 mg/ml ampicilin. Ampicilin resistant colonies are recovered and grown in Lb liquid media with 100 mg/ml ampicilin. Plasmid DNA is purified from the cultures by standard miniprep procedures. The correct construct is verified by the susceptibility for cleavage with Fok I.
      9) Cleavage with Fok I:
         A 5 mg sample DNA is treated with 10 units of Fok I (New England Biolabs) for 60 minutes at 37°C in a 50 ml reaction consisting of 50 mM potassium acetate, 20 mM Tris-acetate (pH 7.9), 10 mM Mg acetate, 1 mM DTT and 100 mg/ml BSA. The DNA is purified as described above.
      10) Klenow Treatment:
         The Fok I digested DNA is treated with 10 units of DNA polymerase I large (Klenow) fragment (New England Biolabs) for 60 minutes at 37°C in a 50 ml reaction consisting of 10 mM Tris-acetate (pH 7.5), 5 mM MgCl₂, 7.5 mM DTT, 1 mM ATP,
         1 mM GTP, 1 mM CTP, 1 mM TTP and 100 mg/ml BSA. The DNA is purified as described above.
      11) Cleavage with Hind III:
         The Klenow-treated DNA is further digested with Hind III and purified as already described.
      12) Preparation of pSPORT Ib for iterative ligation of polyester coding sequences: The plasmid pSPORT Ib is digested with the restriction endonuclease Fok I, which causes the plasmid to become linearized and to have the following termini:
         -5'C-AGC-ACG-AGG-GGA-AAT-CTG-ATG-3'
         -3'-TGCAG-TCG-TGC-TCC-CCT-TTA-GAC-TACATTA-5'
         and
         5'-TAAT-GCATGC-GCG-**CATCC**-A-3'-
         3'-CGTACG-CGC-**GTAGG**-TTCGA-5'-
         Key:
         Bold = Fok I site
         The linearized plasmid is treated with Klenow fragment to fill in the 3' overhangs. The DNA is then cleaved with Hind III. This linearized plasmid is designated pSPORT-La.
   4) First round of ligation of polyester coding sequence into pSPORT-La: In this step, a set of oligonucleotides is ligated into pSPORT-La between the blunt ended 5' termini and the 3' Hind III termini. The oligonucleotides are synthesized with the following sequence: 
5'-AA-TAA-TAA-TAA-TAA-TAA-TAA-TAA-TAA-TAA-TAA-TAA-TAA-TAA-
TAA-TAA-TAA-TAA-TAA-TAA-TAA-TAA-GCATGC-GCG-CATCC-A-3'
VI
3'-TT-ATT-ATT-ATT-ATT-ATT-ATT-ATT-ATT-ATT-ATT-ATT-ATT-ATT-
ATT-ATT-ATT-ATT-ATT-ATT-ATT-ATT-CGTACG-CGC-GTAGG-TTCGA-5'
 Key:
      Bold = Fok I site
      The oligonucleotides are combined, heated to 90°C then allowed to cool slowly to room temperature. During cooling, the oligonucleotides anneal to form a double stranded DNA. The double stranded DNA is ligated into pSPORT-La.
      13) Ligation of oligonucleotide VI into Klenow treated and Hind III digested pSPORT-La:
         The Klenow-treated and Hind III digested pSPORT-La DNA is combined with 1 mg of annealed oligonucleotide VI. The DNA solution is treated with 10 units of T4 DNA ligase in a 100 ml reaction consisting of 50 mM Tris-HCl (pH 7.8), 10 mM DTT, 1 mM ATP and 100 mg/ml BSA at 15°C for 16 hours. The new plasmid, designated pSPORT Ic, has the first segment of the polyester coding region, followed by Fok I and Hind III sites. The sequence is diagramed below (showing only the top strand of the DNA duplex):
         -5-'AGC-ACG-AGG-GGA-AAT-CTG-**ATG**-[TAA]₂₄-Fok I-Hind III-3'- (VII)
         Key:
         Bold = start codon
         Underline = trpA initiator sequence
      12) Transformation and selection of pSPORT Ic:
         The ligation reaction is used to transform competent E. coli DH5-a. The transformed E. coli are selected for the presence of plasmid by growing colonies on Lb agar plates with 100 mg/ml ampicilin. Ampicilin resistant colonies are recovered and grown in Lb liquid media with 100 mg/ml ampicilin. Plasmid DNA is purified from the cultures by standard miniprep procedures. The correct construct is verified by the molecular weight.
      15) Second round of ligation of polyester-coding sequence into pSPORT-Ic: pSPORT-Ic is prepared for ligation of polyester coding sequence as described above for pSPORT-Ib. The resulting linearized plasmid is designated pSPORT-Lb. A new set of oligonucleotides is synthesized which contains sequences for the next section of the polyester coding sequence: 
5'-TAA-TAG-TAA-TAG-TAA-TAG-TAA-TAG-TAA-TAG-TAA-TAG-TAA-TAG-
TAA-TAG-TAA-TAG-TAA-TAG-TAG-TAG-TAG-TAG-TAG-TAG-TAG-TAG-TAG-
TAG--GCATGC-GCG-CATCC-A-3'
VIII
3'-ATT-ATC-ATT-ATC-ATT-ATC-ATT-ATC-ATT-ATC-ATT-ATC-ATT-ATC-
ATT-ATC-ATT-ATC-ATT-ATC-ATC-ATC-ATC-ATC-ATC-ATC-ATC-ATC-ATC-
ATC--CGTACG-CGC-GTAGG-TTCGA-5'
 Key:
         Bold = Fok I site
         The oligonucleotides are combined, heated to 90°C, then allowed to cool slowly to room temperature. During cooling, the oligonucleotides anneal to form a double stranded DNA. The double stranded DNA is ligated into pSPORT-Lb. The resulting new plasmid, designated pSPORT Id, has approximately two thirds of the polyester coding region, followed by Fok I and Hind III sites. The sequence is diagramed below (showing only the top strand of the DNA duplex):
         -5-'AGC-ACG-AGG-GGA-AAT-CTG-**ATG**-[TAA]₂₄-[TAA-TAG]₁₀-
         [TAG]₁₀-Fok I-Hind III-3'- (IX)
         Key:
         Bold = start codon
         Underline = trpA initiator sequence
      16) Third round of ligation of polyester-coding sequence into pSPORT-Id: The plasmid pSPORT-Id is prepared for ligation of the last segment of the polyester coding sequence as described above for pSPORT-Ib. The linearized plasmid is designated pSPORT-Lc. A new set of oligonucleotides is synthesized that contains sequences for the last section of the polyester coding sequence and the STOP codon: 
5'-TAG-TAG-TAG-TAG-TAG-TAG-TAG-TAG-TAG-TAG-TAG-TAG-TAG-TAG-
TAG-TGA-A-3'
X
3'-ATC-ATC-ATC-ATC-ATC-ATC-ATC-ATC-ATC-ATC-ATC-ATC-ATC-ATC-
ATC-ACT-TTCGA-5'
 The oligonucleotides are combined, heated to 90°C, then allowed to cool slowly to room temperature. During cooling, the oligonucleotides anneal to form a double stranded DNA. The double stranded DNA is ligated into pSPORT-Lc. The new plasmid, designated pSPORT Ie, contains the entire polyester transcriptional unit. Transformation and selection of the pSPORT Id and pSPORT Ie plasmids are carried out as described above.
c) Synthesis of polyester-coding RNA:
   1) Preparation of the RNA template: The plasmid pSPORT Ie is linearized with BamH I and subjected to *in vitro* run off transcription.
   2) Cleavage with BamH I:
      A 5 mg sample DNA is treated with 10 units of BamH I (New England Biolabs) for 60 minutes at 37°C in a 50 ml reaction consisting of 150 mM NaCl, 50 mM Tris-HCl (pH 7.9), 10 mM MgCl₂, 1 mM DTT and 100 mg/ml BSA. The resulting linearized DNA is purified as described above.
   3) In vitro Run Off Transcription:
      A 1 mg sample of the linearized BamH I digested DNA is treated with 10 units of SP6 RNA Polymerase (BRL) for 60 minutes at 37°C in a 50 ml reaction consisting of SP6 promoter-primer, 40 mM Tris-HCl (pH 7.9), 6 mM MgCl₂, 2 mM Spermidine-(HCl)3, 1 mM DTT, 0.4 mM rATP, 0.4 mM rGTP, 0.4 mM rCTP, 0.4 mM UTP and 100 mg/ml BSA. The reaction is stopped by the addition of sodium dodecyl sulphate (SDS) and the nucleic acids are purified. The DNA is digested with RNase free DNase and the RNA is purified by extraction and precipitation, as described herein for DNA.

### III) Synthesis of Sup-tRNAphe-lactate and Sup-tRNAphe-glycolate:

tRNA molecules for lactate and glycolate are synthesized using the deamination method. In the present example, Sup-tRNA-lactate and Sup-tRNA-glycolate are produced directly in a fermentation process. These tRNA species are then translated *in vitro* using templates and translation systems as described above.

Genes for yeast tRNA^{gly} and tRNA^{ala} are cloned onto a M13 type phage vector and subjected to site directed mutagenesis. The resulting mutagenized tRNA has new anticodons that recognize stop codons (Sup-tRNA^{gly} and Sup-tRNA^{ala}). In this example, the stop codons chosen for tRNA^{ala} and for tRNA^{gly} are UAA and UAG, respectively. The new tRNA genes are cloned onto an appropriate expression vector downstream of an inducible promoter. These plasmids are used to transform yeast. The resulting recombinant yeast strain is used to produce large quantities of Sup-tRNA^{gly}-glycine and Sup-tRNA^{ala}-alanine

Cultures of the yeast are grown to near confluence. A protein synthesis inhibitor is added and the inducible tRNA genes are activated. The yeast express the tRNA^{gly} and tRNA^{ala}, which are aminoacylated with the appropriate amino acid. The resulting Sup-tRNA^{gly}-glycine and Sup-tRNA^{ala}-alanine are purified by standard techniques. Purified Sup-tRNA^{gly}-glycine and Sup-tRNA^{ala}-alanine are incubated in 0.25 M sodium acetate, 0.01 M magnesium acetate, 1M NaNO₂ at 24°C. The pH is maintained at pH 4.3 with acetic acid. These reaction conditions result in the deamination of the amino acids on the tRNA to the a-hydroxyael analogs as shown in Figure 5. The resulting Sup-tRNAgly-glycolate and Sup-tRNA^{ala}-lactate are purified by column chromatography.

### IV) INITIATOR tRNAf:

Protein synthesis is specifically initiated at the first AUG codon. The process of initiation involves a unique tRNA called tRNAf. This tRNAf is normally aminoacylated with methionine to generate Met-tRNAf. The charged tRNAf is then converted to Formylmethionyl-tRNAf, or fMET-tRNAf. The initiation factors for protein synthesis specifically recognize fMET-tRNAf, and initiate translation using this tRNA at the first AUG codon.

To provide the initiator tRNA for polyester synthesis, a synthetic initiator tRNA is synthesized such that it recognizes the AUG codon, but carries a lactate instead of methionine. A truncated version of the E. coli tRNAf is generated that does not have the CpA dinucleotide at the 3' termini. A synthetic CpA dinucleotide is synthesized and coupled with lactate to form CpA-lactate The CpA-lactate is ligated onto the 3' termini of the truncated tRNAf. The resulting Lactate-tRNAf initiates translation by recognizing the AUG codon; however, translation is initiated with lactate rather than methionine. In the present example, the starting tRNAf must be E. coli so that it will be recognized by the initiation factors in the E. coli *in vitro* translation system.

### V) IN VITRO TRANSLATION OF POLYESTER TEMPLATE:

In the present example, an E. coli *in vitro* translation system is employed. E. coli amino-acyl tRNA transferase cannot amino-acylate yeast tRNA, which in this example is the origin of the Sup-tRNA^{phe}-glycolate. Thus, it will be appreciated that once the Sup-tRNAphe-lactate and Sup-tRNAphe-glycolate participate in a translation cycle, the free Sup-tRNA^{phe} will not be amino-acylated with phenylalanine and thus interfere with polyester synthesis. In addition, the E. coli strain used to generate the *in vitro* translation extract is a recombinant strain that does not express tRNA for the two stop codons used to encode glycolate and lactate. Therefore, native tRNA will not compete with Sup-tRNA^{phe}-glycolate for recognition of stop codons.

The translation extract is used to synthesize polymers in conjunction with the artificial template. Free amino acids, ATP, GTP, Sup-tRNA^{phe}-lactate and Sup-tRNA^{phe}-glycolate, and template are added to the E. coli extract. Translation is terminated by the addition of a detergent, and amide-polyester polymers are purified by column chromatography. The purified polymer is processed by the cleavage steps described earlier to separate the amide from the polyester segment. If desired, the polyester segment is further purified by extraction methods or additional column chromatography.

The RNA template for the polyester is combined with a cell free E. coli translation system. A pool of the Sup-tRNA^{phe}-lactate and Sup-tRNA^{phe}-glycolate is added, along with ATP and GTP. In addition, Lactate-tRNAf or MET-tRNAf is added.

### VI) PURIFICATION AND PROCESSING OF THE POLYESTER:

The polyester is purified from the *in vitro* translation reaction by extraction in methylene chloride followed by further purification by gel permeation chromatography.

Since the polyester of the present example is synthesized with Lactate-tRNAf, no further processing is required.

### EXAMPLE II

### TEMPLATE DIRECTED SYNTHESIS OF A POLYESTER WITH A POLYPEPTIDE LEADER SEQUENCE

**I) DESIGNATION OF POLYESTER SEQUENCE:** In the present example, a polyester having the sequence I (as in Example I) is produced. It differs from the polyester of Example I in that it includes a polypeptide leader sequence, as described below.
**II) DESIGN AND SYNTHESIS OF THE SYNTHETIC GENE:**
   A) The polyester coding region: This section of the template is identical to that described in Example I.
   B) Stepwise construction of the transcriptional unit:
      1) E. coli fusion protein expression vector: Since *in vitro* translation of the synthetic mRNA is carried out in an E. coli cell free system, the initiator region must be recognized by E. coli ribosomes. In this example, the fusion protein expression vector pMAL-p2 (New England Biolabs) is used. This commercially available vector is used to express fusion protein in E. coli. Therefore, all of the necessary initiator sequences are already present, along with the template for the polypeptide leader sequence.
      2) First round of ligation of polyester coding sequence into pMAL-p2: The following oligonucleotides are synthesized: 
5'-TAA-TAA-TAA-TAA-TAA-TAA-TAA-TAA-TAA-TAA-TAA-TAA-TAA-TAA-
TAA-TAA-TAA-TAA-TAA-TAA-TAA-TAA-GCATGC-GCG-CATCC-A-3'
XI
3'-ATT-ATT-ATT-ATT-ATT-ATT-ATT-ATT-ATT-ATT-ATT-ATT-ATT-ATT-
ATT-ATT-ATT-ATT-ATT-ATT-ATT-ATT-CGTACG-CGC-GTAGG-TTCGA-5'
 Key:
         Bold = Fok I site
         Underline = Hind III adaptors
         The oligonucleotides are combined, heated to 90°C, then allowed to cool slowly to room temperature. During cooling, the oligonucleotides anneal to form a double stranded DNA. The double stranded DNA is ligated into pMAL-p2 that has been digested with XmnI and Hind III. The new plasmid, pMAL-p2a, has the first section of the polyester coding sequence ligated in frame with the polypeptide leader sequence.
      3) Second round of ligation of polyester coding sequence into pMAL-p2a: To initiate the second round of template construction, pMAL-p2a is digested with Fok I, treated with Klenow fragment, then digested with Hind III. The linearized version of pMAL-p2a is designated pMAL-p2aL. The following oligonucleotides are synthesized: 
5′-TAA-TAA-TAA-TAA-TAG-TAA-TAG-TAA-TAG-TAA-TAG-TAA-TAG-TAA-
TAG-TAA-TAG-TAA-TAG-TAA-TAG-TAA-TAG-GCATGC-GCG-CATCC-A-3′
XII
3′-ATT-ATT-ATT-ATT-ATC-ATT-ATC-ATT-ATC-ATT-ATC-ATT-ATC-ATT-
ATC-ATT-ATC-ATT-ATC-ATT-ATC-ATT-ATC-CGTACG-CGC-GTAGG-TTCGA-
5′
 The oligonucleotides are combined, heated to 90°C, then allowed to cool slowly to room temperature. During this time the oligonucleotides anneal to form a double stranded DNA. The double stranded DNA is ligated into pMAL-p2aL. The new plasmid, designated pMAL-p2b, has two-thirds of the polyester coding sequence ligated in frame with the polypeptide leader sequence.
      4) Third round of ligation of polyester coding sequence into pMAL-p2a: To initiate the third round of template construction, pMAL-p2b is digested with Fok I, treated with Klenow fragment, then digested with Hind III. The linearized version of pMAL-p2b is designated pMAL-p2bL. The following oligonucleotides are synthesized: 
5'-TAG-TAG-TAG-TAG-TAG-TAG-TAG-TAG-TAG-TAG-TAG-TAG-TAG-TAG-
TAG-TAG-TAG-TAG-TAG-TAG-TAG-TAG-TAG-TAG-TAG-TGA-GCATGC-GCG-
CATCC-A-3'
XIII
3'-ATC-ATC-ATC-ATC-ATC-ATC-ATC-ATC-ATC-ATC-ATC-ATC-ATC-ATC-
ATC-ATC-ATC-ATC-ATC-ATC-ATC-ATC-ATC-ATC-ATC-ACT-CGTACG-CGC-
GTAGG-TTCGA-5'
 The oligonucleotides are combined, heated to 90°C, then allowed to cool slowly to room temperature. During this time the oligonucleotides anneal to form a double stranded DNA. The double stranded DNA is ligated into pMAL-p2aL. The new plasmid, designated pMAL-p2C, has the complete polyester coding sequence ligated in frame with the polypeptide leader sequence.
   c) Synthesis of Polyester-Coding RNA:
      1) Preparation of the RNA template: pMAL-p2c is used to transform E. coli strain DH5a. The E. coli is grown to stationary phase and IPTG is added to initiate transcription of the RNA for the polypeptide-polyester fusion protein. After 1-2 hours the E. coli are collected and total RNA is purified by standard methods. The RNA template is purified from the total RNA by affinity chromatography using a column that has covalently attached oligonucleotides that anneal to the template sequence.
**III) SYNTHESIS OF SUP-tRNAphe-LACTATE AND SUP-tRNAphe-GLYCOLATE:**
   The tRNA molecules for lactate and glycolate are synthesized as described in Example I.
**IV) IN VITRO TRANSLATION OF POLYESTER TEMPLATE:**
   The RNA template is prepared as described in Example I. The RNA template for the polyester is combined with a cell free E. coli translation system. A pool E. coli tRNA, Sup-tRNA^{phe}-lactate and Sup-tRNA^{phe}-glycolate and ATP and GTP are added. The translation reaction is terminated with the addition of SDS.
**V) PURIFICATION AND PROCESSING OF THE POLYESTER:**
   The polypeptide-polyester fusion polymer is purified from the *in vitro* translation reaction by affinity chromatography. The polypeptide region of the fusion polymer encoded by the pMAL-p2 vector is the maltose binding protein. The fusion polymer is separated from the *in vitro* reaction mixture by passing the sample through an amylose column. The polymer binds to the amylose by virtue of the maltose binding protein segment. The fusion polymer is eluted from the column with free maltose.
   The polypeptide portion of the fusion polymer is cleaved from the polyester segment by treatment with cyanogen bromide.

The claims which follow identify embodiments of the invention additional to those described in detail above.

## Claims

1. A medical device or article comprising a synthetic polymer which is biocompatible, the synthetic polymer comprising a lactic acid and glycolic acid polyester copolymer in which each successive lactic acid and glycolic acid monomeric unit in the polyester copolymer has been individually and specifically determined.

2. The medical device or article of claim 1 which is absorbable.

3. The medical device or article of claim 1 or 2, additionally comprising one or more medicinal agents.

4. A method of making a medical device or article wherein a lactic acid and glycolic acid polyester copolymer is synthesized by a process comprising:
a. altering Supp.tRNA so as to accept lactic acid and glycolic acid, to produce Supp.tRNA^{1ac} and Supp.tRNA^{gly} respectively,
b. preparing an mRNA sequence including triplet codons recognized by the Supp.tRNA^{1ac} and Supp.tRNA^{gly} of step (a);
c. providing an expression translation system;
d. introducing into the system of step (c) the products of steps (a) and (b) under conditions which allow the production of lactic acid glycolic acid polyester copolymer; and
e. collecting the copolymer produced in step (d).

5. A process for the manufacture of monofilament sutures comprising: melt extruding polyester resin to provide a monofilament; stretching the solidified monofilament at a temperature above ambient in water or other suitable liquid or gaseous medium; to provide a stretched monofilament; wherein the polyester resin is comprised of a synthetic polymer which is bioresorbable, wherein the synthetic polymer comprises a lactic acid and glycolic acid polyester copolymer in which each successive lactic acid and glycolic acid monomeric unit in the polyester copolymer has been individually and specifically determined.

6. A medical device or article, or a structure, made by the process of claim 4 and/or claim 5.

7. A suture as claimed in claim 6, being an armed suture.
